# FASCICULE DE BREVET EUROPEEN

(11) **EP 0 566 450 B1**
(45) Date de publication et mention de la délivrance du brevet: **19.02.1997**
(21) Numéro de dépôt: 93400898.8
(22) Date de dépôt: 06.04.1993
(51) Int. Cl.: A61N 5/02, A61N 5/04

(54) **Dispositif applicateur d'hyperthermie par micro-ondes dans un corps certain**
Mikrowellenvorrichtung zur Induzierung von Hyperthermie in einem Körper
Micro-waves device inducing hyperthermia in a body

(30) Priorité: 08.04.1992 FR 9204298
(43) Date de publication de la demande: 20.10.1993
(73) Titulaire: INSTITUT NATIONAL DE LA SANTE ET DE LA RECHERCHE MEDICALE (INSERM), 75654 Paris Cédex 13 (FR); UNIVERSITE DES SCIENCES ET TECHNIQUES DE LILLE FLANDRES-ARTOIS (LILLE I), 59655 Villeneuve d'Ascq Cedex (FR)
(72) Inventeur: Sozanski, Jean-Pierre, F-59239 Thumeries (FR); Chive, Maurice, F-59650 Villeneuve d'Ascq (FR); Moschetto, Yves, F-59320 Ennetieres en Weppes (FR)
(74) Mandataire: Fréchède, Michel

(56) Documents cités:
- EP-A- 0 370 890
- EP-A- 0 462 302

## Description

La présente invention est relative à un dispositif applicateur d'hyperthermie par micro-ondes dans un corps certain.

Les applicateurs d'hyperthermie micro-ondes sont actuellement utilisés en thermothérapie, pour le traitement de l'adénome de la prostate par exemple.

Dans un tel cas, un cathéter de type Foley, c'est-à-dire un cathéter comportant un ballonet de positionnement gonflable est introduit dans l'urètre du patient, le positionnement étant effectué par gonflage du ballonet, ces cathéters devant nécessairement grouper des fonctions de refroidissement du cathéter, en vue de stabiliser les parois environnantes des tissus à traiter, et de mesure des températures correspondantes.

Les documents EP-A-0462302 et EP-A-0370890 décrivent des dispositifs selon l'état de la technique.

Après utilisation d'un cathéter dans les conditions précitées, celui-ci doit être, soit jeté au rebut, soit stérilisé, en vue d'une nouvelle utilisation. Cette dernière opération de stérilisation n'est pas facilement réalisable car, en raison de la complexité des cathéters actuels, l'opération de stérilisation précitée est susceptible de détériorer ces derniers, en particulier en ce qui concerne des éléments, fragiles, destinés à mesurer la température qui leur est associée. En conséquence, une perte non négligeable en résulte, les praticiens et personnels utilisant ces types de cathéter préférant, pour des raisons évidentes de sécurité, procéder à la mise au rebut des cathéters précités après leur première utilisation.

La présente invention a pour objet de supprimer les inconvénients précités par la mise en oeuvre d'un dispositif applicateur d'hyperthermie dans lequel, bien qu'un cathéter classique soit utilisé, les fonctions de mesure de température et de thermostatisation (ou refroidissement) du cathéther et de l'antenne applicateur sont totalement séparées de ce dernier.

Un autre objet de la présente invention est également, compte tenu de la séparation des fonctions précitées de la fonction proprement dite de cathéter, la mise en oeuvre d'un dispositif applicateur d'hyperthermie utilisant un cathéter particulièrement simple et peu onéreux.

Un autre objet de la présente invention est également, l'utilisation, dans le dispositif applicateur d'hyperthermie selon l'invention, d'un cathéter amovible, ce qui, compte tenu de la simplicité du cathéter utilisé, permet sans inconvénient de réaliser une mise au rebut de celui-ci, après une ou plusieurs utilisations.

Un autre objet de la présente invention est enfin, compte-tenu de la séparation des fonctions, la mise en oeuvre d'un mouvement alterné programmable et/ou rotatif du cathéter applicateur d'hyperthermie dans le cathéter amovible afin d'augmenter la zone de chauffage et/ou de moduler la dose thermique de traitement.

Le dispositif applicateur d'hyperthermie par micro-ondes dans un corps certain, objet de la présente invention, comprend une antenne filaire alimentée en énergie micro-onde, ou jouant, sur commutation, le rôle d'une sonde radiométrique de réception pour la mesure de température par radiométrie micro-onde. Il est remarquable en ce qu'il comporte un manchon permettant de thermostater l'antenne filaire au moyen d'un fluide de thermostatisation. Un cathéter de type Foley est monté, de manière amovible, de façon à englober l'ensemble ainsi formé par rapport à une ouverture d'introduction dans le corps certain. Un module de connexion est prévu, ce module permettant l'interconnexion, tant du point de vue radio-électrique de l'antenne filaire que du point de vue hydraulique du manchon et du cathéter.

Le dispositif applicateur d'hyperthermie par micro-ondes objet de la présente invention trouve application non seulement à la thermothérapie mais également, d'une manière beaucoup plus générale, au traitement thermique contrôlé de corps certains.

Une description plus détaillée de l'applicateur d'hyperthermie par micro-ondes objet de la présente invention sera donnée ci-après à l'aide des dessins dans lesquels :
- la figure 1 représente, selon une vue en coupe longitudinale partielle, le dispositif applicateur d'hyperthermie,
- la figure 1b représente en coupe longitudinale partielle le dispositif applicateur d'hyperthermie associé à un dispositif alterné, en vue de moduler la dose thermique de traitement,
- la figure 2a représente, selon une vue en coupe longitudinale partielle, une première variante de réalisation d'un dispositif applicateur selon l'invention,
- la figure 2b représente, selon une vue en coupe longitudinale partielle, une deuxième variante de réalisation d'un dispositif applicateur selon l'invention,
- la figure 2c représente une vue en coupe selon le plan de coupe AA du mode de réalisation représenté en figure 2a ou 2b,
- la figure 3a représente une variante de mise en oeuvre d'un module de connexion particulièrement adapté à la réalisation du dispositif applicateur objet de la présente invention,
- la figure 3b représente une vue de face d'un élément de connexion mâle constituant le module de connexion représenté en 3a,
- la figure 3c représente une vue en coupe longitudinale d'un élément de connexion femelle constituant le module de connexion représenté en figure 3a.

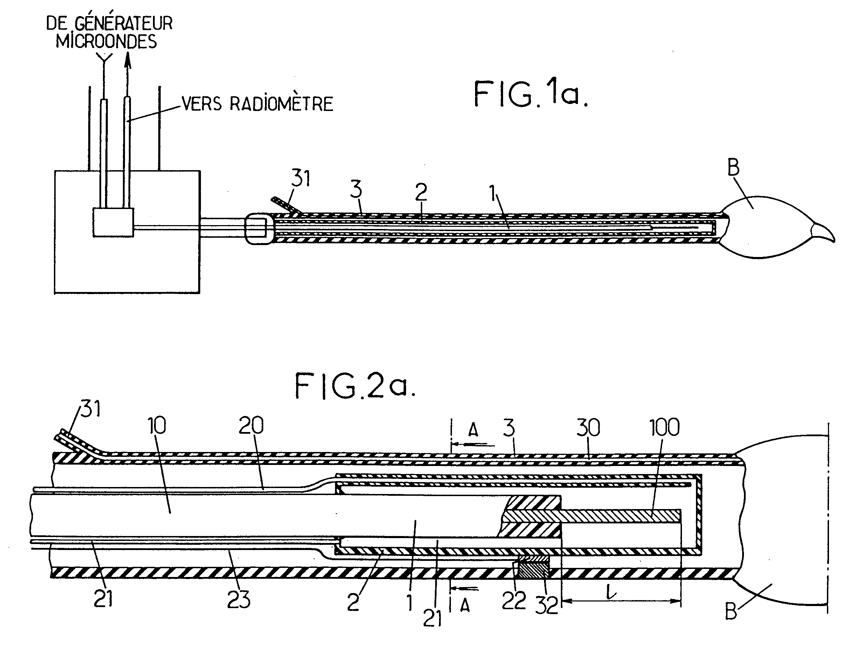

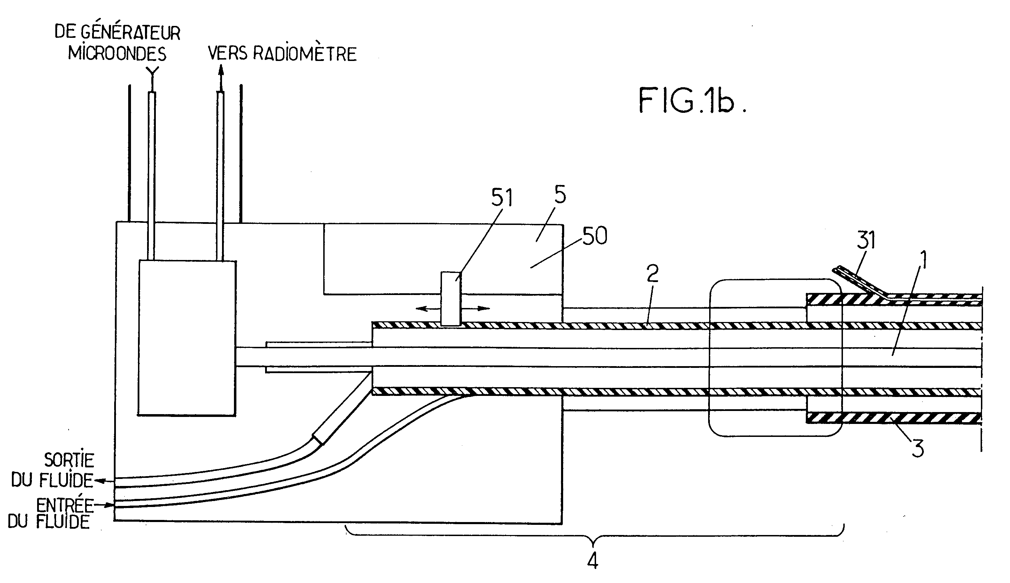

Le dispositif applicateur d'hyperthermie par micro-ondes objet de la présente invention sera décrit de manière plus détaillée en premier lieu en liaison avec la figure 1.

Sur la figure précitée, on pourra remarquer à l'observation de celle-ci que le dispositif applicateur d'hyperthermie par micro-ondes objet de la présente invention comprend une antenne filaire, notée 1, alimentée en énergie micro-ondes ou jouant, sur commutation, le rôle d'une sonde radiométrique de réception pour la mesure de température par radiométrie micro-onde. Des indications supplémentaires seront données relativement à la commutation précitée ultérieurement dans la description, bien que la commutation proprement dite ne fasse pas partie de l'objet de la présente invention.

Ainsi qu'il apparaît à l'observation de la figure 1, le dispositif selon l'invention comporte un manchon 2 permettant de thermostater l'antenne filaire 1 au moyen d'un fluide de thermostatisation. On notera que le fluide de thermostatisation peut être constitué par de l'eau par exemple ou par un fluide caloporteur non corrosif tel que une huile au silicone, par exemple.

Le dispositif applicateur d'hyperthermie par micro-ondes objet de la présente invention comporte en outre un cathéter 3 de type Foley monté de manière amovible de façon à englober l'ensemble formé par le manchon 2 et l'antenne filaire 1. De manière classique, le cathéter 3 comprend un ballonet B en extrémité de cathéter, ce cathéter pouvant être constitué par exemple par un cathéter béquillé, c'est-à-dire comportant une extrémité incurvée, et une prise 31, reliée à une pompe non représentée au dessin, de façon à assurer le gonflage du ballonet B par l'intermédiaire d'une canalisation non représentée sur la figure 1. Ainsi, de manière classique, le cathéter 3 permet de positionner l'ensemble ainsi formé par rapport à une ouverture d'introduction dans le corps certain de l'ensemble précité.

En outre, le dispositif applicateur d'hyperthermie par micro-ondes objet de la présente invention comprend un module 4 de connexion permettant d'assurer l'interconnexion, tant du point de vue radio-électrique de l'antenne filaire 1, que du point hydraulique du manchon 2 et du cathéter.

Un dispositif ou module 5 d'entraînement du manchon 2 peut être prévu, lequel permet d'effectuer un mouvement alterné programmable du manchon 2 dans le cathéter 3. Ce déplacement peut être programmé en fonction du temps (modulation de la dose thermique) et/ou en fonction du parcours (longueur et positionnement du balayage effectué). Le module d'entraînement 5 peut être constitué par exemple par un moteur pas à pas, 50, programmable permettant grâce à une tige d'entraînement, 51, de soumettre le manchon 2 , et l'antenne montés souples, c'est-à-dire l'extrémité rayonnante de celle-ci, à un mouvement alterné de translation, ce qui permet de moduler la dose ou degré d'hyperthermie appliquée. L'amplitude du mouvement est de 2 à 4 cm par exmple et de plus est réglable en temps.

D'une manière générale, on rappellera que le cathéter 3 de type Foley peut être réalisé, par exemple, par un cathéter classique en caoutchouc, alors que le manchon 2 permettant de thermostater l'antenne pourra être réalisé par un tube cylindrique souple en matériau de type PVC ou nylon, la longueur du tube cylindique souple constituant le manchon étant alors fonction de la partie active de l'antenne.

En outre, le dispositif applicateur d'hyperthermie par micro-ondes objet de la présente invention comprend un module 4 de connexion permettant d'assurer l'interconnexion tant du point de vue radio-électrique de l'antenne filaire 1 que du point de vue hydraulique du manchon et du cathéter. On notera bien entendu que le module 4 de connexion permet d'assurer l'interconnexion de l'antenne filaire 1 vers des circuits venant du générateur micro-ondes et, d'autre part, vers des circuits allant vers un radiomètre, conformément au mode opératoire précédemment décrit, par exemple, dans la demande de brevet français numéro 89 10148 au nom de la demanderesse.

Un premier mode de réalisation du dispositif objet de la présente invention sera décrit en liaison avec la figure 2a.

La figure 2a représente une vue en coupe longitudinale, vue partielle, du dispositif objet de la présente invention.

D'une manière générale, on notera que l'antenne filaire 1 est constituée par un câble coaxial 10 dont l'extrémité, constituant extrémité rayonnante 100, est dénudée sur une longueur l, laquelle dépend de la fréquence de l'énergie micro-onde choisie pour le chauffage. A titre d'exemple non limitatif, la longueur l peut être prise égale à 4 cm environ pour une fréquence de 915 MHz.

Selon la figure 2a, le manchon 2 est alors un manchon court recouvrant au plus le câble coaxial 10 en son extrémité sur une longueur au plus égale à 3 fois la longueur dénudée l. On notera alors que l'extrémité du manchon 2 opposée à l'extrémité rayonnante de l'antenne filaire peut alors être avantageusement scellée par collage au moyen d'une résine araldite par exemple à la gaine du câble coaxial 10 de façon à assurer l'étanchéité de la zone thermostatée matérialisée par l'intérieur du manchon 2.

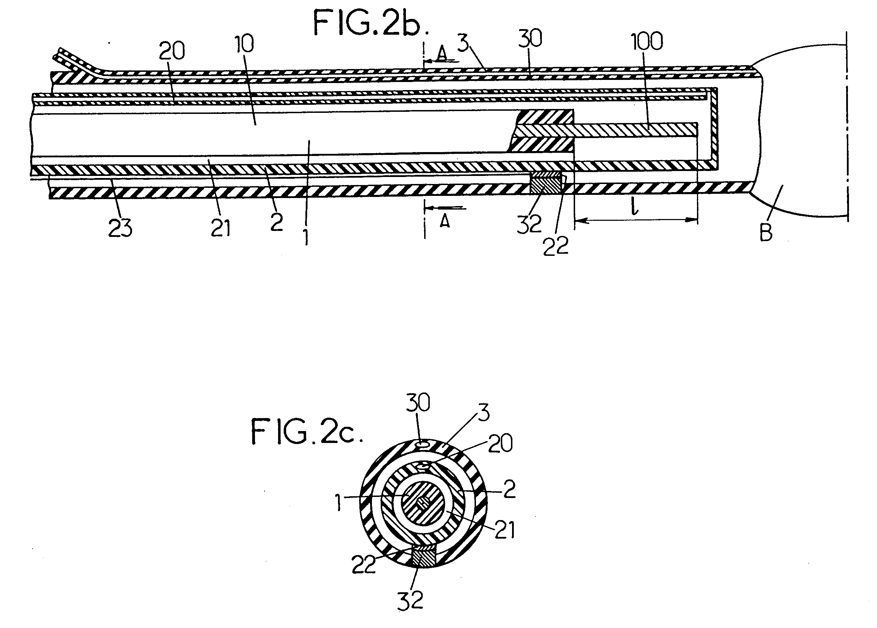

Selon le mode de réalisation de la figure 2b au contraire, le manchon 2 est constitué par un manchon long recouvrant la totalité du câble coaxial 10 sur toute la longueur de celui-ci jusqu'au module d'interconnexion 4, ainsi qu'il sera décrit ultérieurement dans la description. Dans les deux cas, c'est-à-dire dans le cas du mode de réalisation de la figure 2a ou 2b, le manchon 2 est constitué par un tube en matériau plastique diélectrique ainsi que mentionné précédemment, ce tube comportant dans sa paroi latérale au moins une lumière 20 jouant le rôle de canalisation d'amenée du fluide hydraulique de thermostatisation. Le retour du fluide hydraulique est assuré par l'interstice 21 présent entre le câble coaxial 10 et la paroi interne du manchon 2.

On notera que dans le cas où le tube constitutif du manchon 2 est un tube cylindrique, la lumière 20 est ménagée le long d'une ligne génératrice de la paroi du tube précité, de façon à aboucher au niveau de l'extrémité rayonnante 100 de l'antenne filaire.

On notera également que, dans le cas où le manchon est un manchon court, ainsi que représenté en figure 2a, la lumière 20 est prolongée au-delà du manchon, c'est-à-dire au-delà de l'extrémité scellée à la gaine du câble coaxial 10, par l'intermédiaire d'une canalisation constituée par un tube 20 matériau plastique, lequel prolonge la lumière 20 précitée et, pour cette raison, porte la même référence.

Il en est de même pour l'interstice 21, lequel est prolongé de manière avantageuse par un tube 21, le tube 21 et le tube 20 jouant le rôle respectivement de canalisations de retour de fluide de thermostatisation, respectivement d'amenée de ce même fluide. Les tubes 20 et 21 peuvent alors avantageusement être fixés par collage sur la gaine du câble coaxial, par exemple. On notera que les tubes en matériau plastique 20 et 21 assurant le prolongement des lumières correspondantes peuvent avantageusement être réalisés par des tuyaux de diamètre intérieur d'environ 0,5 mm et de diamètre extérieur de 0,8 mm.

Ainsi qu'on l'a en outre représenté en figures 2a et 2b, le manchon 2 peut comprendre de manière avantageuse sur sa paroi externe au voisinage de l'extrémité rayonnante 100 de l'antenne filaire une cellule thermo-couple 22 permettant de mesurer la température externe de la paroi du corps certain à traiter. Bien entendu, un fil de connexion 23 permet de relier alors la cellule thermocouple 22 au module de connexion 4 afin de permettre de relever le signal délivré par la cellule de thermocouple 22 et ainsi mesurer la température correspondante.

Afin de réaliser une mesure de température fidèle de la paroi du corps certain à traiter, le cathéter 3 de type Foley peut comprendre, ainsi que représenté en figures 2a et 2b, dans toute l'épaisseur de sa paroi interne et au voisinage de son extrémité, c'est-à-dire du ballonnet B, un pont thermique 32. Ce pont thermique est destiné, après mise en place du cathéter sur le manchon, à entrer en contact direct avec la cellule thermo-couple 22 disposée sur la paroi externe du manchon 2.

Le pont thermique 32 peut, par exemple, être réalisé sous forme d'une pastille de résine thermodurcissable chargée par des particules métalliques conductrices de la chaleur. Les particules métalliques pourront être constituées par des particules de cuivre électrolytique ou d'argent.

Sur la figure 2c, on a représenté une vue en coupe selon le plan de coupe AA des figures 2a et 2b du dispositif applicateur d'hyperthermie par micro-ondes objet de la présente invention. Sur la figure 2c précitée, on constate la dispositition sensiblement concentrique de l'antenne filaire 1 constituée par le câble coaxial 10 du manchon 2, puis du cathéter 3. En ce qui concerne les dimensions d'encombrement des différents éléments constitutifs précités, on indiquera que le câble coaxial 10, c'est-à-dire le câble muni de sa gaine diélectrique dans la partie non dénudée et d'un blindage écran électro-statique externe plus, le cas échéant, d'une couche de protection de celui-ci par une pellicule de matériau plastique, présente un diamètre compris entre 2,5 à 3 mm, le manchon 2 présente un diamètre de l'ordre de 4 à 5 mm, et enfin le cathéter 3 présente un diamètre externe inférieur à 9 mm. On indiquera en outre que la paroi du manchon 2, lorsque celui-ci est constitué par un matériau silicone, par exemple, peut présenter une épaisseur comprise entre 5/10 mm et 1 mm.

En ce qui concerne le cathéter 3, on notera que différentes tailles de cathéter peuvent être utilisées, et en particulier des cathéters de diamètre externe 5,7 ; 6 ; 6,3 mm correspondant aux normes désignées par les appellations "French 17, French 18 et French 19".

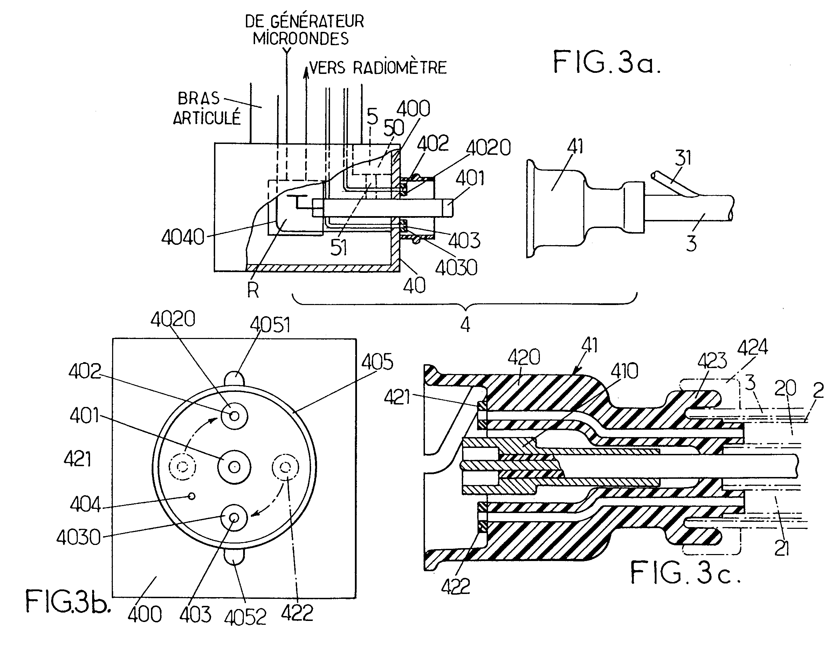

Une description plus détaillée du module de connexion 4 sera maintenant donnée en liaison avec les figures 3a à 3c.

Selon la figure 3a précitée, le module de connexion 4 comprend avantageusement un bloc de connexion mâle 40 formant boîtier de connexion. Le bloc de connexion mâle 40 comporte, sur une face, 400, du boîtier, un premier connecteur micro-ondes, 401, destiné à assurer l'interconnexion de l'antenne filaire 1 et un deuxième connecteur, 402, destiné à assurer l'interconnexion de la conduite d'amenée du fluide hydraulique de thermostatisation et un troisième connecteur, 403, destiné à assurer au contraire l'interconnexion de la conduite de retour du fluide de thermostatisation. Le bloc de connexion mâle, 40, comporte en outre à l'intérieur du boîtier des circuits d'alimentation correspondants.

On indiquera à titre d'exemple non limitatif que le premier connecteur radio-électrique, 401, peut être relié vers les circuits provenant du générateur micro-ondes d'une part, et vers un circuit radiomètre d'autre part, par un relais micro-ondes à faibles pertes hyperfréquences permettant de commuter l'antenne applicateur soit vers le générateur de chauffage soit vers le radiomètre. Le mode de réalisation de l'interconnexion du premier connecteur micro-ondes, 401, vers le générateur ou vers le radiomètre ne fait pas partie de l'objet de la présente invention.

Ainsi qu'on l'a représenté en outre sur la figure 3a, le module de connexion 4 comprend un élément de connexion femelle, noté 41, lequel est solidaire du cathéter, du manchon et du câble coaxial formant antenne filaire. On comprend bien sûr que le qualificatif mâle ou femelle réservé à l'élément de connexion 40, respectivement 41, peut être interverti sans sortir du cadre de l'objet de la présente invention.

D'une manière générale, le premier connecteur micro-ondes, 401, peut être réalisé par un connecteur verrouillable indépendant. Le deuxième, 402, et le troisième, 403, connecteurs de l'élément de connexion mâle, 40, pouvant alors être constitués avantageusement par des orifices de connexion disposés symétriquement par rapport à l'axe central du premier connecteur micro-ondes, 401. On notera alors que les orifices de connexion constitutifs des deuxième, 402, et troisième, 403, connecteurs sont munis de joints d'étanchéité, 4020, 4030, lesquels sont de manière classique constitués par des bagues de type O-ring.

La figure 3b représente ainsi une vue de la face, 400, du boîtier constitutif du bloc de connexion mâle 40, cette face 400 comportant respectivement le premier connecteur radio-électrique, 401, en position centrale, le deuxième, 402, et le troisième, 403, connecteurs formés par les orifices correspondants et leurs joints d'étanchéité, 4020, 4030. En outre, on notera en position intermédiaire par rapport aux deuxième et troisième connecteurs, 402, 403, l'existence d'un plot, 404, de type plot électrique, lequel est relié par un élément conducteur, 4040, vers les circuits de traitement, ce plot de contact, 404, étant destiné à recevoir le contact d'un élément correspondant disposé au niveau de l'élément de connection femelle, 41, et relié par exemple au fil 23 de liaison avec la cellule thermocouple 22. Ces derniers éléments ne seront pas représentés au dessin afin de ne pas surcharger inutilement ces derniers.

On notera enfin que le bloc de connexion 40 comprend en outre sur la face 400 précitée une couronne circulaire externe, notée 405, centrée sur le premier connecteur radioélectrique, 401, et munie de deux tétons diamétralement opposés, notés 4051, 4052. La collerette, 405, et les tétons, 4051, 4052, sont destinés à recevoir et à assurer l'interconnexion du bloc de connexion mâle 40 avec l'élément de connexion femelle 41, ainsi qu'il sera décrit ci-après en liaison avec la figure 3b précitée et avec la figure 3c.

Selon la figure 3c précitée, le module de connexion 4 comprend également un élement de connexion femelle, 41, cet élément comportant un premier connecteur femelle, noté 410, constitué par un connecteur radio-électrique et destiné à assurer l'interconnexion de l'antenne filaire 1 et du câble coaxial 10 avec le premier connecteur radio-électrique, 401.

En outre, ainsi que représenté sur la figure 3c précitée, l'élément de connexion femelle, 41, comprend un deuxième connecteur femelle, 420, lequel est coaxial au premier, 410, et est destiné à assurer l'interconnexion de la conduite d'amenée, 20, respectivement de la conduite de retour, 21, du fluide hydraulique de thermostatisation au deuxième, 402, respectivement troisième, 403, connecteur du bloc de connexion mâle, 40.

A titre d'exemple non limitatif, on indiquera que le premier connecteur femelle radio-électrique, 410, peut être constitué par un corps métallique dans lequel est monté classiquement le câble coaxial 10. Au contraire, le deuxième connecteur femelle, 420, peut, lui, être réalisé par un corps de connecteur en matériau plastique, moulé par exemple. Ainsi, le plot de contact non représenté sur la figure 3c destiné à venir au contact du plot de contact, 404, correspondant du bloc de connection 40, peut-il être relié directement par un fil noyé dans le corps de connecteur femelle 41, et non représenté au dessin. On notera à titre d'exemple non limitatif que de manière avantageuse, le corps du premier connecteur femelle 410 et le corps du deuxième connecteur femelle 420 sont montés libres à coulissement et à rotation l'un par rapport à l'autre. On notera en outre que le corps du deuxième connecteur femelle, 420, comporte des orifices, 4210, 4220, reliés par des canaux correspondants à la partie arrière du corps de connecteur femelle 41, les orifices précités étant bien entendu munis de joints d'étanchéité, 4210, 4220, constitués par des joints O-ring, par exemple, de la même manière que dans le cas du corps de connecteur mâle, 40. De préférence, les orifices, 421 et 422, sont situés de manière sensiblement symétriques et diamétralement opposés par rapport à l'axe longitudinal du premier connecteur femelle 410. On notera enfin que le corps du deuxième connecteur femelle 41 est également muni d'ouvertures d'insertion et de verrouillage ménagées sur la paroi du corps de connecteur femelle 420 et portant la référence 425. On notera que, de manière particulièrement avantageuse, ces ouvertures de guidage et de verrouillage, 425, sont ménagées de façon telle que leur ouverture sur le bord de la collerette formant le corps de connecteur femelle 41 est décalée d'un quart de tour par rapport à la position des orifices 421 et 422 correspondants.

Ainsi, le premier connecteur femelle, 410, peut-il être interconnecté sur le premier connecteur mâle, 401, par vissage, le deuxième connecteur femelle, 420, ayant été déplacé en translation de façon à rendre le premier connecteur femelle, 410, apparent et accessible, puis, le cathéter 3 ayant été installé sur la partie arrière du connecteur femelle, 41, le deuxième connecteur femelle, 420, est alors interconnecté sur le deuxième, 402, et le troisième, 403, connecteur mâle par rotation quart de tour. Les joints d'étanchéité respectifs, 4210, 4220, sont alors amenés par la rotation quart de tour en vis-à-vis des joints d'étanchéité, 4020, respectivement 4030, du bloc de connexion 40, ainsi que représenté en figure 4b. La fixation du deuxième connecteur femelle, 420, est alors assurée par l'intermédiaire des tétons, 4051, 4052, ménagés sur la collerette, 405, précédemment décrite.

Afin d'assurer une bonne mise en place du cathéter 3 au niveau de la partie arrière du connecteur femelle, 420, celui-ci peut comporter avantageusement sur la face externe du deuxième connecteur femelle, 420, une bague, 423, de positionnement relatif et de blocage du cathéter 3 de type Foley par rapport au manchon 2. En outre, une bague de fixation définitive par vissage, 424, peut être prévue de façon à assurer le maintien du cathéter pendant la durée de l'opération. On notera bien sûr que les tubes constitutifs des canalisations des amenées respectivement du retour du fluide de thermostatisation, 20 et 21, peuvent préalablement avoir été fixés sur la partie arrière du connecteur et du deuxième connecteur femelle, 420, ainsi que représenté en figure 3c.

On notera en outre que, le premier connecteur femelle 410 étant monté libre en translation, avant connexion, dans le corps de connecteur femelle 41, le premier connecteur micro-ondes 401 de l'élément de connexion 40 peut également être monté à coulissement en translation, car relié au relais micro-ondes R par un câble coaxial souple, ce qui permet au module 5 d'entraînement d'assurer l'entraînement de l'ensemble, le manchon pouvant être un manchon court.

On a ainsi décrit un dispositif applicateur d'hyperthermie par micro-ondes dans un corps certain particulièrement performant dans la mesure où la structure de ce type d'applicateur d'hyperthermie permet de séparer les fonctions de mesure de température et de thermostatisation du cathéter, et de l'antenne applicateur au cathéter de type Foley utilisé dans le dispositif objet de la présente invention étant seulement dévolue une fonction de positionnement, ce qui permet, après utilisation du cathéter, d'une structure particulièrement simple, d'envisager la mise au rebut de celui-ci après une ou voire plusieurs utilisations.

En outre, on notera que la mise en oeuvre d'un module d'interconnexion particulièrement adapté permet d'assurer un branchement de façon permanente de toutes les connexions électriques et fluidiques, en ce qui concerne le fluide de thermostatisation, ce qui permet bien entendu de supprimer l'effet "spaghetti" des interconnexions peu apprécié des praticiens.

## Revendications

1. Dispositif applicateur d'hyperthermie par microondes dans un corps certain, comprenant un cathetér de type Foley et une antenne filaire alimentée en énergie micro-onde ou jouant, sur commutation, le rôle d'une sonde radiométrique pour la mesure de température par radiométrie micro-onde, caractérisé en ce que ledit dispositif comporte trois éléments constitutifs amovibles associés pour réaliser la fonctionnalité du dispositif :
- un module (4) de connexion permettant l'interconnexion tant du point de vue radio-électrique de l'antenne filaire (1) que du point de vue hydraulique d'un manchon et d'un cathéter,
- un manchon (2) permettant de thermostater ladite antenne filaire, au moyen d'un fluide de thermostatisation,
- ledit cathéter (3) de type Foley, monté de manière amovible de façon à englober l'ensemble formé par le manchon (2) et ladite antenne filaire (1), ledit cathéter permettant de positionner l'ensemble ainsi formé par rapport à une ouverture d'introduction dans le corps certain, et étant la seule partie jetable pour assurer une bonne hygiène de la séance d'hyperthermie.

2. Dispositif selon la revendication 1, caractérisé en ce que ladite antenne filaire (1) étant constituée par un câble coaxial (10) dont l'extrémité, constituant extrémité rayonnante (100), est dénudée sur une longueur l, ledit manchon est un manchon court recouvrant au plus ledit câble coaxial en son extrémité sur une longueur au plus égale à trois fois la longueur dénudée l.

3. Dispositif selon la revendication 1, caractérisé en ce que ladite antenne filaire (1) étant constituée par un câble coaxial (10) dont l'extrémité, constituant extrémité rayonnante (100), est dénudée sur une longueur l, ledit manchon est un manchon long recouvrant la totalité du câble coaxial, jusqu'au module d'interconnexion.

4. Dispositif selon l'une des revendications 1 à 3, caractérisé en ce qu'il comprend un module (5) d'entraînement programmable du manchon (2) et de l'antenne (1) en mouvement relatif dans le cathéter (3).

5. Dispositif selon l'une des revendications précédentes, caractérisé en ce que ledit manchon (2) est constitué par un tube en matériau diélectrique, ledit tube comportant dans sa paroi latérale au moins une lumière (20) jouant le rôle de canalisation d'amenée du fluide hydraulique de thermostatisation, le retour dudit fluide hydraulique étant assuré par l'interstice (21) présent entre ledit câble coaxial (10) et la paroi interne du manchon.

6. Dispositif selon la revendication 5, caractérisé en ce que ledit tube étant un tube cylindrique, ladite lumière (20) est ménagée le long d'une ligne génératrice de la paroi dudit tube de façon à aboucher au niveau de l'extrémité rayonnante (100) de l'antenne filaire.

7. Dispositif selon l'une des revendications précédentes, caractérisé en ce que ledit manchon (2) comprend sur sa paroi externe, au voisinage de ladite extrémité rayonnante, une cellule thermocouple (22) permettant de mesurer la température externe de la paroi du corps certain à traiter.

8. Dispositif selon la revendication 7, caractérisé en ce que ledit cathéter (3) de type Foley comprend dans toute l'épaisseur de la paroi interne, au voisinage de son extrémité, un pont thermique (32), ledit pont thermique étant destiné, après mise en place du cathéter sur le manchon, à entrer en contact direct avec ladite cellule thermocouple (22) disposée sur la paroi externe du manchon.

9. Dispositif selon la revendication 8, caractérisé en ce que ledit pont thermique (32) est formé par une pastille de résine thermodurcissable chargée par des particules métalliques.

10. Dispositif selon l'une des revendications précédentes, caractérisé en ce que ledit module de connexion (4) comprend un bloc de connexion mâle (40) formant boîtier de connexion, ledit bloc de connexion comportant, sur une face (400) du boîtier, un premier connecteur micro-ondes (401) destiné à assurer l'interconnexion de ladite antenne filaire, un deuxième connecteur (402), destiné à assurer l'interconnexion de la conduite d'amenée du fluide hydraulique de thermostatisation, un troisième connecteur (403) destiné à assurer l'interconnexion de la conduite de retour du fluide de thermostatisation, ledit bloc de connexion mâle (40) comportant à l'intérieur du boîtier des circuits d'alimentation correspondants.

11. Dispositif selon la revendication 10, caractérisé en ce que ledit premier connecteur micro-onde (401) est un connecteur verrouillable indépendant, le deuxième (402) et le troisième (403) connecteur étant constitués par des orifices de connexion disposés symétriquement par rapport à l'axe central du premier connecteur (401) et munis de joints d'étanchéité (4020, 4030).

12. Dispositif selon la revendication 10 ou 11, caractérisé en ce que ledit module de connexion (4) comprend un élément de connexion femelle (41) solidaire du cathéter, du manchon et du câble coaxial formant ladite antenne filaire, ledit élément de connexion femelle comportant :
- un premier connecteur femelle (410) destiné à assurer l'interconnexion de ladite antenne filaire,
- un deuxième connecteur femelle (420) coaxial au premier et destiné à assurer l'interconnexion de la conduite d'amenée (20) respectivement la conduite de retour (21) du fluide hydraulique de thermostatisation.

13. Dispositif selon la revendication 12, caractérisé en ce que le premier connecteur femelle (410) est interconnecté sur le premier connecteur mâle (401) par vissage, et le deuxième connecteur femelle (420) est interconnecté sur le deuxième (402) et le troisième (403) connecteur mâle par rotation quart de tour.

14. Dispositif selon la revendication 13, caractérisé en ce que ledit deuxième connecteur femelle (420) comporte, en outre, sur sa face externe, une bague (423) de positionnement relatif et de blocage du cathéter (3) de type Foley par rapport audit manchon.

## Claims

1. Device for application of hyperthermia in a particular body using microwaves, comprising a Foley-type catheter, a wire antenna supplied with microwave energy or, upon switching, acting as a radiometric probe for measuring temperature using radiowave radiometry, characterised in that the said device includes three associated removable constituent elements for producing the functionality of the device:
- a connection module (4) allowing the interconnection both of the wire antenna (1), from the radiofrequency point of view, and of a sleeve and a catheter, from the hydraulic point of view,
- a sleeve (2) enabling the said wire antenna to be thermostatted by means of a thermostatting fluid,
- said Foley-type catheter (3) removably mounted so as to encompass the assembly formed by the sleeve (2) and the said wire antenna (1), the said catheter enabling the assembly thus formed to be positioned in relation to an opening for insertion into the particular body, and being the sole disposable part in order to ensure good hygiene of the hyperthermia session.

2. Device according to Claim 1, characterised in that, the said wire antenna (1) being constituted by a coaxial cable (10), the end constituting the radiating end (100) of which is stripped over a length l, the said sleeve is a short sleeve covering at most the said coaxial cable at its end over a length not exceeding three times the stripped length l.

3. Device according to Claim 1, characterised in that, the said wire antenna (1) being constituted by a coaxial cable (10), the end constituting the radiating end (100) of which is stripped over a length l, the said sleeve is a long sleeve covering the whole coaxial cable, right up to the interconnection module.

4. Device according to one of the Claims 1 to 3, characterised in that it comprises a programmable module (5) for driving the sleeve (2) and the antenna (1) in a relative motion in the catheter (3).

5. Device according to one of the preceding Claims, characterised in that the said sleeve (2) is constituted by a tube made from dielectric material, the said tube including, in its lateral wall, at least one elongate hole (20) acting as a duct for inlet of the hydraulic thermostatting fluid, the said hydraulic fluid returning via the gap (21) existing between the said coaxial cable (10) and the inner wall of the sleeve.

6. Device according to Claim 5, characterised in that the said tube being a cylindrical tube, the said elongate hole (20) is made along a line which is a generatrix of the wall of the said tube so as to join up with the radiating end (100) of the wire antenna.

7. Device according to one of the preceding Claims, characterised in that the said sleeve (2) comprises, on its outer wall in the vicinity of the said radiating end, a thermocouple cell (22) making it possible to measure the outer temperature of the wall of the particularly body to be treated.

8. Device according to Claim 7, characterised in that the said Foley-type catheter (3) comprises, throughout the thickness of the inner wall, in the vicinity of its end, a thermal bridge (32), the said thermal bridge being intended, after the catheter has been installed on the sleeve, to come into direct contact with the said thermocouple cell (22) placed on the outer wall of the sleeve.

9. Device according to Claim 8, characterised in that the said thermal bridge (32) is formed by a pad of thermosetting resin filled with metal particles.

10. Device according to one of the preceding Claims, characterised in that the said connection module (4) comprises a male connection unit (40) forming a connection case, said connection unit including, on one face (400) of the case, a first microwave connector (401) intended to interconnect the said wire antenna, a second connector (402) intended to interconnect the conduit for inlet of the hydraulic thermostatting fluid, a third connector (403) intended to interconnect the conduit for return of the thermostatting fluid, the said male connection unit (40) including corresponding supply circuits inside the case.

11. Device according to Claim 10, characterised in that the said first mmicrowave connector (401) is an independent lockable connector, the second (402) and third (403) connectors being constituted by connection orifices arranged symmetrically in relation to the central axis of the first connector (401) and fitted with leaktight seals (4020, 4030).

12. Device according to Claim 10 or 11, characterised in that the said connection module (4) comprises a female connection element (41) rigidly attached to the catheter, to the sleeve and to the coaxial cable forming said wire antenna, the said female connection element including:
- a first female connector (410) intended to interconnect the said wire antenna,
- a second female connector (420) coaxial with the first and intended to interconnect, respectively, the inlet conduit (20) and the return conduit (21) for the hydraulic thermostatting fluid.

13. Device according to Claim 12, characterised in that the first female connector (410) is interconnected to the first male connector (401) by screwing and the second female connector (420) is interconnected to the second (402) and the third (403) male connector by a rotation of a quarter of a turn.

14. Device according to Claim 13, characterised in that the said second female connector (420) furthermore includes, on its outer face, a ring (423) for relative positioning and for locking of the Foley-type catheter (3) in relation to the said sleeve.

## Patentansprüche

1. Vorrichtung zum Applizieren von Hyperthermie mittels Mikrowellen in einem bestimmten Körper, umfassend einen Foley-Katheter und eine Drahtantenne, welche mit Mikrowellenenergie versorgt ist oder, nach Umschaltung, die Rolle einer Radiometriesonde für die Messung der Temperatur durch Mikrowellenradiometrie spielt, dadurch gekennzeichnet, daß
die Vorrichtung drei wesentliche Bestandteile umfaßt, welche lösbar vereinigt sind, um die Funktionalität der Vorrichtung zu realisieren:
- ein Verbindermodul (4), welches die Verbindung sowohl der Drahtantenne (1) in radioelektrischer Hinsicht als auch einer Hülse und eines Katheters in hydraulischer Hinsicht ermöglicht,
- eine Hülse (2), welche die Thermostabilisierung der Drahtantenne mittels eines Thermostabilisierungsfluids ermöglicht,
- den Foley-Katheter (3), welcher lösbar derart angeordnet ist, daß er die aus der Hülse (2) und der Drahtantenne (1) gebildete Einheit umgibt, wobei es der Katheter ermöglicht, die so gebildete Einheit bezüglich einer Einführungsöffnung in dem bestimmten Körper zu positionieren und wobei der Katheter zur Sicherstellung guter Hygiene bei der Hyperthermiesitzung das einzige Wegwerfteil ist.

2. Vorrichtung nach Anspruch 1, dadurch gekennzeichnet, daß die Drahtantenne (1) durch ein Koaxialkabel (10) gebildet ist, dessen Ende, welches das strahlende Ende (100) bildet, auf einer Länge l blank ist und die Hülse eine kurze Hülse ist, welche das Koaxialkabel an seinem Ende höchstens über eine Länge bedeckt, welche höchstens gleich dem Dreifachen der blanken Länge l ist.

3. Vorrichtung nach Anspruch 1, dadurch gekennzeichnet, daß die Drahtantenne (1) durch ein Koaxialkabel (10) gebildet ist, dessen Ende, welches das strahlende Ende (100) bildet, auf einer Länge l blank ist und die Hülse eine lange Hülse ist, welche die Gesamtheit des Koaxialkabels bis zu dem Verbindermodul bedeckt.

4. Vorrichtung nach einem der Ansprüche 1 bis 3, dadurch gekennzeichnet, daß sie ein Modul (5) zum programmierbaren Antrieb der Hülse (2) und der Antenne (1) zur Relativbewegung in dem Katheter (3) umfaßt.

5. Vorrichtung nach einem der vorangehenden Ansprüche, dadurch gekennzeichnet, daß die Hülse (2) durch eine Röhre aus dielektrischem Material gebildet ist, wobei die Röhre in ihrer Seitenwandung wenigstens einen Einlaß (20) aufweist, der die Rolle der Zuführungsleitung für das Hydraulikfluid zur Thermostabilisierung spielt, wobei der Rücklauf des Hydraulikfluids durch den zwischen dem Koaxialkabel (10) und der Innenwandung der Hülse vorhandenen Zwischenraum (21) gesichert ist.

6. Vorrichtung nach Anspruch 5, dadurch gekennzeichnet, daß die Röhre eine zylindrische Röhre ist und der Einlaß (20) entlang einer Mantellinie der Wandung der Röhre derart vorgesehen ist, daß er sich im Bereich des strahlenden Endes (100) der Drahtantenne öffnet.

7. Vorrichtung nach einem der vorangehenden Ansprüche, dadurch gekennzeichnet, daß die Hülse (2) an ihrer äußeren Wandung in der Nähe des strahlenden Endes eine Thermoelementzelle (22) umfaßt, welche es erlaubt, die Außentemperatur der Wandung des zu behandelnden bestimmten Körpers zu messen.

8. Vorrichtung nach Anspruch 7, dadurch gekennzeichnet, daß der Foley-Katheter (3) über die gesamte Dicke der Innenwandung in der Nähe seines Endes eine Wärmebrücke (32) umfaßt, wobei die Wärmebrücke dazu bestimmt ist, nach Aufbringen des Katheters auf der Hülse in direkten Kontakt mit der Thermoelementzelle (22) zu treten, welche an der Außenwandung der Hülse angeordnet ist.

9. Vorrichtung nach Anspruch 8, dadurch gekennzeichnet, daß die Wärmebrücke (32) aus einer Pastille aus mit metallischen Partikeln beladenem warmaushärtbarem Harz gebildet ist.

10. Vorrichtung nach einem der vorangehenden Ansprüche, dadurch gekennzeichnet, daß das Verbindermodul (4) einen Steckerverbinderblock (40) umfaßt, welcher das Verbindergehäuse bildet, wobei der Verbinderblock an einer Seite (400) des Gehäuses einen zur Sicherstellung der Verbindung der Drahtantenne bestimmten ersten Mikrowellenverbinder (401), einen zur Sicherstellung der Verbindung der Zuführungsleitung für Hydraulikfluid zur Thermostabilisierung vorgesehenen zweiten Verbinder (402) und einen zur Sicherstellung der Verbindung der Rückführungsleitung des Fluids zur Thermostabilisierung bestimmten dritten Verbinder (403) umfaßt, wobei der Steckerverbinderblock (40) im Inneren des Gehäuses entsprechende Versorgungskreise umfaßt.

11. Vorrichtung nach Anspruch 10, dadurch gekennzeichnet, daß der erste Mikrowellenverbinder (401) ein unabhängiger verriegelbarer Verbinder ist und der zweite (402) und der dritte (403) Verbinder durch Verbindungsöffnungen gebildet sind, welche bezüglich der Zentralachse des ersten Verbinders (401) symmetrisch angeordnet sind und mit Dichtungen (4020, 4030) versehen sind.

12. Vorrichtung nach Anspruch 10 oder 11, dadurch gekennzeichnet, daß das Verbindermodul (4) ein Buchsenverbinderelement (41) umfaßt, welches mit dem Katheter, der Hülse und dem die Drahtantenne bildenden Koaxialkabel verbunden ist, wobei das Buchsenverbinderelement umfaßt:
- einen ersten Buchsenverbinder (410), welcher dazu bestimmt ist, die Verbindung der Drahtantenne sicherzustellen,
- einen zweiten Buchsenverbinder (420), welcher zu dem ersten koaxial ist und dazu bestimmt ist, die Verbindung der Zuführungsleitung (20) bzw. der Rückführungsleitung (21) des Hydraulikfluids zur Thermostabilisierung sicherzustellen.

13. Vorrichtung nach Anspruch 12, dadurch gekennzeichnet, daß der erste Buchsenverbinder (410) auf dem ersten Steckerverbinder (401) durch Verschraubung angebunden ist und der zweite Buchsenverbinder (420) auf dem zweiten (402) und dem dritten (403) Steckerverbinder durch eine Vierteldrehung angebunden ist.

14. Vorrichtung nach Anspruch 13, dadurch gekennzeichnet, daß der zweite Buchsenverbinder (420) ferner an seiner Außenseite einen Ring (423) zur relativen Positionierung und zur Festlegung des Foley-Katheters (3) bezüglich der Hülse umfaßt.
